# EUROPEAN PATENT SPECIFICATION

(11) **EP 0 489 258 B1**
(45) Date of publication and mention of the grant of the patent: **07.02.1996**
(21) Application number: 91118621.1
(22) Date of filing: 31.10.1991
(51) Int. Cl.: C22B 3/00

(54) **A bio-metallurgical process in which bio-oxidation of mineral compounds is produced**
Biometallurgisches Verfahren zur Bio-Oxidation von anorganischen Verbindungen
Procédé bio-métallurgique pour le bio-oxydation de composés minéraux

(30) Priority: 07.11.1990 AR 318330
(43) Date of publication of application: 10.06.1992
(73) Proprietor: LEACHING S.R.L., Santiago (CL); SHELL CHILE S.A. COMERCIAL E INDUSTRIAL, Santiago (CL)
(72) Inventor: Panos, Nora Hilda, Barrio Rivadavia,5400 San Juan (AR)
(74) Representative: Dost, Wolfgang, Dr.rer.nat., Dipl.-Chem.

(56) References cited:
- WO-A-84/02355
- US-A- 4 729 788
- METALLURGICAL TRANSACTIONS B vol. 20B, no. 6, December 1989, WARRENDALE, PA, US pages 773 - 779; A. BALLESTER ET AL.: 'MICROBIOLOGICAL LEACHING OF COPPER FROM LEAD MATTES'
- METADEX, AN 85(8):42-1084, P. T. Malakhova et al "Increasing the Efficiency of Leaching Copper From the Kalmakyr Deposits.", Tsvetn. Met. 1985, (2), 21-23.

## Description

The present invention refers to a process in which mineral compounds, contained in mineral ores or concentrates and constituting substrates for microorganisms, are bio-oxidized to allow solubilization and separation thereof.

### TECHNICAL FIELD OF THE INVENTION

Biotechnology of metals is the science of extracting metals from minerals, concentrates, rocks and solutions by the action of microorganisms or their metabolites at normal pressure and at a temperature of 5 to 90° C. One of its areas of technological development is Biohydrometallurgy which refers to the oxidation of sulphide minerals, elemental sulphur, ferrous iron and a number of reduced metals by acidophilus microorganisms, turning them into soluble, easily separable compounds. Optimization of this technology could advantageously compete with the conventional processes of extractive metallurgy

It has been proved that by bacterial leaching it is possible to oxidize the following sulfides: pyrite and marcasite (FeS₂), pyrrhotite (FeS), chalcopyrite (CuFeS₂), bornite (Cu₅FeS₄), covellite (CuS), chalcocite (Cu₂S), tetrahedrite (CuₑSb₂S₇), enargite (3Cu₂S.As₂S₅), molybdenite (MoS₂), sphalerite (ZnS), arsenopyrite (FeAsS), realgar (AsS), orpi- ment (As₂S₃), cobaltite (CoAsS), pentlandite (Fe,Ni)₉ S₈, violarite (Ni₂FeS₄), bravoite (NiFe)S₂, millerite (NiS), poly- dymite (Ni₃S₄), antimonite (Sb₂S₃), marmatite (ZnS), galena (PbS), geocronite Pbₛ(Sb, As₂)S₈, Ga₂S₃ and CuSe among others. Tt is known that microbial species oxidize insoluble sulphide minerals into soluble sulphates either directly or indirectly. In the case of direct oxidation, the destruction of the crystalline structure of the sulphide mineral takes place by the enzymatic systems of the acting microorganisms. The indirect oxidation of sulphide minerals is due to the action of the ferric ion (Fe³⁺), which, in turn, is a product of the bacterial oxidation of ferrous iron and iron-containing sulphide minerals.

We shall analyze the chemistry of pyrite biological oxidation via the most probable reaction:

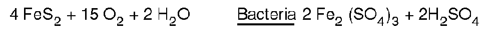

The above reaction illustrates the direct bacterial oxidation of pyrite. The resulting ferric sulphate, in turn, oxidizes pyrite, forming ferrous sulphate and elemental sulphur: 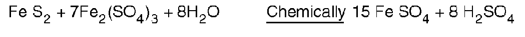 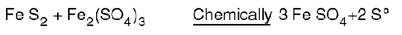 Ferrous iron and sulphur undergo bacterial oxidation:

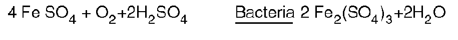

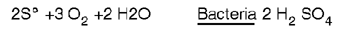

In the case of chalcocite (CU2S) it has been proved that cuprous ion (Cu¹+) and sulphide (S²⁻) are oxidized by the microbian enzymatic systems to Cu²⁺, S° y SO 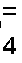 respectively.

By similar mechanisms, the bacterial oxidation of a wide spectrum of sulphide minerals is possible.

Thiobacillus ferrooxidans and related bacteria oxidize the uranous ion according to the following reaction:

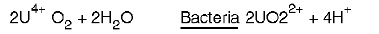

The leading role in uranium leaching is played by ferric iron. Fe³+ oxidizes U⁴+ to U⁶+ which is solubilized in sulfuric acid solutions.

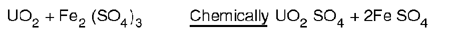

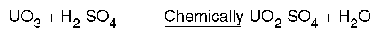

The bacteria regenerate Fe³+ by oxidation of Fe²⁺ or Fe S₂.

By reactions similar to the above, a wide variety of mineral compounds can be oxidized. The most important microorganisms in biohydrometallurgy are presented in Table I.

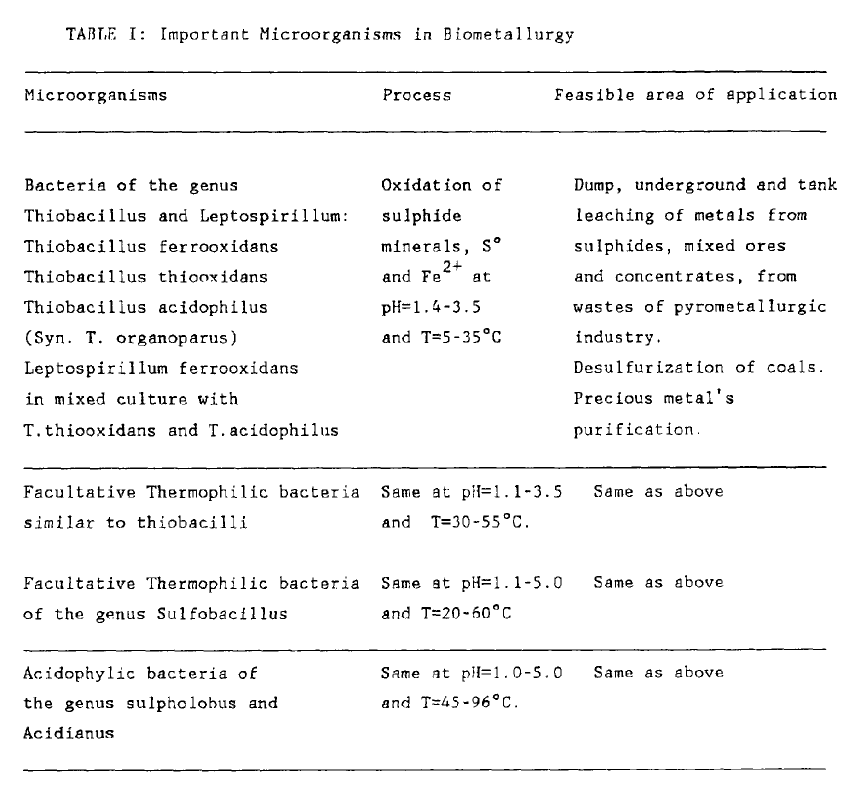

As indicated in Table I, the main areas of application of the biohydrometallurgy processes are : metals leaching, coal desulphurization and precious metals purification. These areas will be briefly discussed hereinbelow:

### Leaching of Metals

At present, the microbial leaching of metals takes place by different processes that depend on the scale and characteristics of the mineral involved

In-situ microbial leaching may be considered as a specialized underground extraction system consisting in the microbiologically enhanced dissolution of metal values from run-of-mine ores with grades ranging from above the cut-off grade to the so-called submarginal or submilling grades. Leach solutions are injected into-and percolate through-the rock mass. When the dissolution of the desired metal values is achieved, the solutions are collected and pumped to the metal recovery plant.

Microbial dump leaching may be defined as a "metal scavenging" method employed for recovering metal values from lean ores, usually the submarginal-grade over-burden of the open-pit mining operations, i.e. that part of the ore body containing rock with grade below the cut-off and which must be removed in order to enable access to the richer parts of the mineralization. These rocks are accumulated in dumps located in the vicinity of the open-pit. The top of the dump is irrigaled with leach solulions containing microorganisms These solutions percolale through the broken rock mass, solubilizing the metal values. The pregnant solutions flow out of the bottom of the dump and are finally collected in ponds and then pumped to the metal recovery plants.

Microbial heap leaching, is a method in which the crushed ore is piled up in regular layers on appropriately prepared areas The heap is a truncated pyramid. The controlling dimension of size is the height of the heap which is related with the graduation of the mineral. The top part of the heap is irrigated with leach solutions containing microorganisms and percolate continuously through the mineral.

Microbial tank leaching is a process whereby metals are leached from ores and concentrates in Pachuca tanks, reactors or conditioning tanks where the pulp formed by the mineral and the leach solution, once inoculated, is stirred and aerated in a thermostated system.

In the biotechnological practice, the dilution of the pulp expressed as the liquid to solid rate mass contained in a given mass of pulp ranges from 4 to 10.

It should be pointed out that even though the different leaching processes developed and put into practice so far have distinctive characteristics, all have a common feature: the ores or concentrates are suspended, flooded and/or subjected to percolation with aqueous solutions. in such a way that the microorganisms are confined to an aqueous environment.

### Coal desulphurization

Coal contains elemental sulphur in variable quantities and mainly as pyrite form (FeS₂). The combustion of coal results in the conversion of the existing sulphur to SO₂, which pollutes the atmosphere causing acid rains with the consequent damages to vegetation, animals and human health. In order to keep appropriate levels of sulphur dioxide in the atmosphere in areas where coal is burned in great scale, low-sulphur coals should be exploited, generally with a total sulphur content below 1 to 1,5%.

Isolation of T ferrooxidans from acid drainages of coal mines, generated interest due to its potential to desulphurize coal by oxidation of sulphur and pyritic minerals.

Microbial leaching of sulphur compounds from coal has been practiced along similar guidelines and under the criteria developed for microbial leaching of metals, that is, the microorganisms must act in an aqueous environment.

Several microorganisms have proved to be effective in coal desulphurization according to conventional techniques. However the process cannot be practiced at industrial scale under such conditions, essentially due to the long processing time and high processing volumes required as a consequence of the low microbial activity.

### Precious metals purification by the liberation of sulphide minerals

It has been demonstrated that when concentrates containing pyrite, arsenopyrite and finely dispersed gold, are subjected to bioleaching prior to cyanidation, most of the sulphide minerals are dissolved and the gold yield is substantially increased by subsequent cyanidation

### Considerations about the optimization of biometallurgical technology

This technology has arised worldwide interest because of its potential advantages over the conventional extractive metallurgical processes:
Low energy consumption
Low chemical reagents consumption
Low investment cost
It is a clean process which does not pollute the environment.
Allows the economical exploitation of low grade deposits.

However in the present state of development, the application of this technology requires long processing times, from several months to years in order to obtain acceptable recoveries. The leaching velocity is lowand this is attributable to the low multiplication velocity of the intervening bacteria.

The processing time constitutes, by itself, a significant technical-economical barrier for the purposes of industrial application.

Also, the low leaching velocity requires operating with large masses of mineral that, in general, are subject to climatic variations, preventing the precise control of the systems which become fluctuating and erratic and result in variable and unpredictable processing times

The study of physiological characteristics, conditions of growth and development of microorganisms related to oxidation of melallic compounds, applied to the solution of the above mentioned technological problems constitutes an important area of investigation encompassing the principles on which the present invention is based

### Description of the Invention

### Introduction

The present invention refers to a biometallurgical process in which mineral compounds contained in mineral ores or concentrates and constituting substrates for microorganisms are bio-oxidized, dissolved and separated. More particularly, the present invention refers to a process that considerably increases the microbial oxidation velocity of mineral compounds. The essential principle of this process is based on the discovery of the bioleaching bacteria behavior with respect to water.

The process of invention is characterized by the following steps
a) conditioning the mineral ore or the concentrate with the quantity of acid which is determined before-hand as the minimum volume that ensures the total and homogenous acidification of the substrate and having a concentration which ensures neutralization of the mineral ore or the concentrate, prevents compaction and provides an environment for microbial development, or contacting the mineral ore or the concentrate with acid vapours so as to homogeneously acidify the substrate while introducing the minimum possible amount of water required to provide an effective coating of the mineral ore or concentrate into the system;
b) adding a microbial inoculum capable of oxidating the mineral compound of interest, or enriching the mineral ore's own microbial flora, either simultaneously with or independently from step a);
c) enabling the spontaneous or induced loss of the excess of water that may be present in the system by evaporation or by dehydrating with flowing air until the thermodynamically available water is sufficiently low for obtaining the bio-oxidation products in solid state, said bio-oxidation products, in turn, constituting microbial colonies; and
d) separating the bio-oxidation products

Preferred embodiments of the invention are disclosed in sub-claims 2 to 9.

The first stage of interaction of bioleaching microorganisms with a solid inorganic substrate consists in their attachment to the surface, whereupon the substrate being oxidized is attacked biochemically. Attachment is specific to the mineral compounds which offer a source of energy, but such attachment is not frequent and does not always occur in the systems so far tried. The conditions which allow or facilitate a stable and efficient attachment, enabling the bacteria to transform the substrate and multiplicate quickly, had not been explained heretofore.

From the physiological characterization and development conditions hereinafter described, it is clear that these microorganisms have a definite hydrophobic character In other words, the water, or at least the water levels in conventional systems, make difficult the stable attachment of cells to the substrates.

The phenomenons that take place when the cells and the surface of mineral compounds interact or the mechanism of destruction of the sulphide mineral lattice are not clear. Although there are different theories, it is generally, believed that enzymatic mechanisms are involved in this interaction. In such case, the intervening enzymes must not be diluted or washed out from the reacting surface.

The culture and bio-oxidation of mineral compounds in the conditions described below, were carried out by using the strains indicated in Table II, by way of example and not limitation. Some of these strains were isolated from minerals from the Argentine deposits "Bajo de la Alumbrera" and "Campana Mahuida".

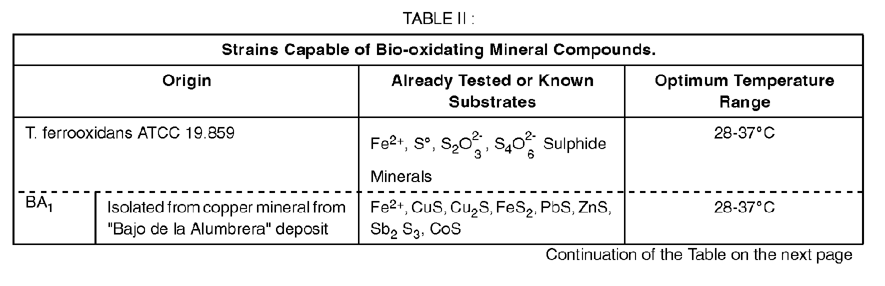

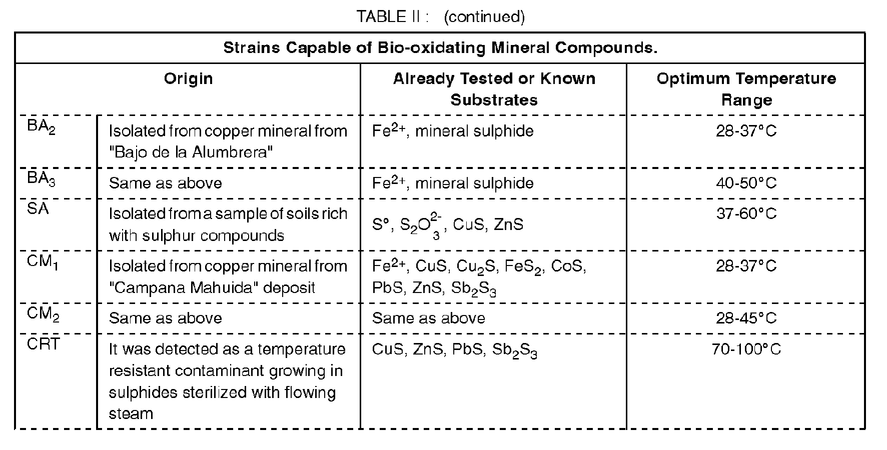

The invention will now be described in the chronological order of the studies and ideas that, lead to its fundamentals, with reference to the accompanying figures.

### Brief Description of the Figures

Figure 1 shows colonies on the dehydrated, thinnest edge of a plate with an agarized ferrous medium. The arrow indicates the inoculation place
Figures 2, 3, 4 and 6 show colonies obtained on dehydrated agarized ferrous medium.
Figure 5 shows colonies obtained in a salt deposit on the glass of a plate. The salts derive from the dehydration of 3cm³ of a concentrated ferrous liquid medium (without gelling agent).
Figure 7 shows schematically a star-shaped colony similar to that indicated with an arrow in Figure 6, in which: (a) designates the centre, (b) the border, (c) the zone between centre and border
Figures 8, 9 and 10 are photographs obtained by scanning microscope corresponding to the centre of a colony indicated with (a) in the schematic drawing of Figure 7
Figures 11, 12 and 13 are photographs obtained by scanning microscope corresponding to the zone of a colony indicated with (c) in the schematic drawing of Figure 7
Figures 14, 15, 16, 17 and 18 are photographs obtained by scanning microscope corresponding to the border of a colony, indicated with (b) in the schematic drawing of Figure 7.
Figure 19 (a) shows the typical tracks of a bacterial surface translocation mechanism, by which bacteria move in groups, called "social gliding". It was produced in a film of analytic grade ferrous sulphate deposited on a plate glass.
Figure 19 (b) shows the colonies obtained in a film of salts containing, in addition to ferrous sulphate, other salts required for bacterial development.
Figure 20 shows blue colonies of a crystalline appearance obtained in acidified synthetic CuS by inoculating the BA2 strain It was incubated at 30°C.
Figure 21 shows a bacterial development associated with light blue crystals in a plate with acidified synthetic CuS. Tt was obtained by inoculating the plate centre with a concentrated suspension of the BA₃ strain It was cultivated at 37°C keeping the plate half open.
Figure 22 shows a bacterial development associated with light blue crystals in a plate with acidified synthetic CuS that was inoculated with the CRT strain. It was incubated at 85°C.
Figure 23 shows colonies of the CM₁ strain associated to soluble iron compounds obtained by using, as substrate, a natural pyrite specimen of high purity, crushed to -100 mesh It was incubated at 30°C.
Figure 24 shows a bacterial development associated to the white colour of zinc sulphate, obtained by using an acidified sphalerite concentrate as substrate. It was inoculated with the ATCC 19.859 strain and incubated at 30°C.
Figure 25 illustrates a plate prepared in the same way as the one on Figure 24. It was dehydrated until 90% of the water added during the acidification, was lost. It was inoculated with a liquid inoculum in the place indicated with the arrow.
Figure 26 shows a bacterial development associated to zinc sulphate, in the inoculation place indicated with the arrow. It was inoculated with the CRT strain, from a solid culture. It was incubated at 96°C keeping the plate halfway open.
Figure 27 shows the development of the BA2 strain, using a natural specimen of Sb₂S₃ as substrate. It was incubated at 30°C.
Figure 28 shows the red colonies obtained by inoculating acidified synthetic cobalt sulphide (CoS) with different strains.
Figure 29 shows light blue colonies obtained by inoculating the BA₁ and CM₁ strains in an acidified concentrate comprising chalcocite (Cu₂S) and enargite (3Cu₂S.As₂S₅) as predominant specimens of copper. 11 was incubated at 30°C keeping the plates open
Figure 30 are photographs from the same plates shown in Figure 29, which were taken at a shorter distance.
Figure 31 (a) shows a mineral ore triturated to 0.64 cm (1/4 inch), comprising chalcocite (Cu₂S) as predominant specimen of copper, not subjected (left) and subjected (right) to bio-oxidation by the CM₁ strain. 11 was incubated at 37°C for sixteen hours keeping the plate open. Figure 31 (b) shows the mineral ore subjected to bio-oxidation.
Figure 32 shows photographs of the same colonized and bio-oxidated mineral ore of Figure 31, taken at a shorter distance.
Figure 33 shows the development of the CM₂ strain in a copper mineral crushed to -0,15 mm (-100 mesh) and acidified, which contains 2,1% of chalcocite. It was incubated at 37°C.
Figure 34 shows the development of the CM₂ strain, in the plate area, which was first dehydrated. The arrow indicates the inoculation place. The substrate was the same as in Figure 33.
Figure 35 is a schematic drawing of microscopic observations obtained from the suspension of a bacterial colony in a liquid medium.

### Preliminary Studies of the Growth in Ferrous Agarized Medium

T. ferrooxidans is the bacterium most commonly used in Biometallurgy. Although microorganisms like T ferrooxidans, have, as energetic substrates, numerous insoluble sulphur compounds, in addition to ferrous iron, the solubility of ferrous iron has encouraged its use in agarized media for laboratory cultures.

The culture of T. ferrooxidans in solid agarized medium, in order to obtain colonies, has posed many problems

Several solid ferrous media have been designed so far. All these media support the growth of colonies However, the colonies are small, slow growing (between one and six weeks) and sometimes with non-repetitive results. These difficulties have been attributed to low bacterial mulliplicalion velocily. Besides, agar or the hydrolysis products of the agarose, used as gelling agents, are thought to inhibit growth.

Observations on the obtention of colonies in agarized ferrous medium led to establish bio-oxidation conditions for sulphur compounds which are explained below.

Petri plates prepared with a conventional ferrous medium and 0,5% agarose, and inoculated with the strains ATCC 19.859, BA₁ and BA2, were cultivated at 30°C and observed every six to eight hours by stereomicroscopy For a period of forty days approximately, there was no evidence of growth. On the day the colonies appeared, the beginning of growth was observable by stereomicroscopy, and after a few hours, colonies 0,5-1 mm in diameter were clear to direct observation. If a forty-day period was required to obtain colonies due to an intrinsically low bacterial multiplication velocity, it follows that growth must have been progressive

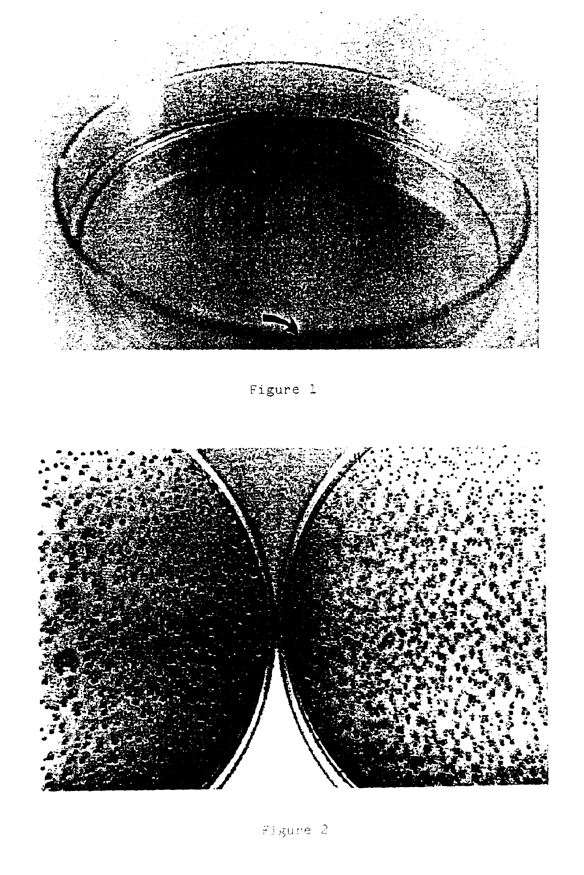

Petri plates placed on a slightly inclined plane were prepared with a ferrous agarized medium in order to obtain a thickness gradient of the agarized culture medium. Thus the culture medium is thickest at one edge of the plate and thinnest at the diametrically opposite edge. The plates were inoculated by touching with a loop holding a liquid inoculum a location on the thickest edge, as indicated with an arrow in Figure 1. At the third or fourth day, colonies were formed on the thinnest edge which is diametrically opposite tothe inoculation place, as shown in Figure 1. There was no evidence of development prior to the day in which colonies were formed. In the case shown in Figure 1, some colonies were formed even on the thin film of medium deposited over the side wall. It must be taken into account that the thinner is a gel, the quicker it is dehydrated.

A great number of tests were made varying agar or agarose concentrations and analyzing the growth according the above guidelines. It is concluded that agar or agarose does not govern the adhesion of these bacteria at the inoculation place, as it generally happens with other bacteria

The essential condition for the formation of colonies is the adhesion of the cells to the agarized medium. Everything happened as if the conditions that allow such adhesion were to be achieved in due time.

The conditions that may vary spontaneously with time in a solid agarized medium containing approximately 95% of water, are the loss of water by evaporation and the resulting increase of concentration of the component salts

Tests were carried out varying the concentrations of the component salts according to wide gradients, without observing a meaningful effect on the time of apparition of the colonies, and without any effect whatsoever on the adhesion of the cells at the inoculation place. Everything indicated that adhesion of the cells to the substrate requires a very low water content.

In tests with plates carrying equal volumes of culture medium, evenly distributed all over the plate, the dehydration degree was increased by subjecting the plates to a laminar flow hood and/or by keeping the plates at 30°C for the spontaneous loss of water prior to inoculation. A considerable decrease in the appearance time of the colonies was observed. Using techniques combining the above-mentioned strategies with the addition of chemical agents that are known to be compatible solutes in biological osmoregulatory systems, it was possible to obtain colonies in twelve to twenty-four hours. The addition of polyethylenglycol to decrease the water activity and the addition of surfactants, also improve the growth in agarized media.

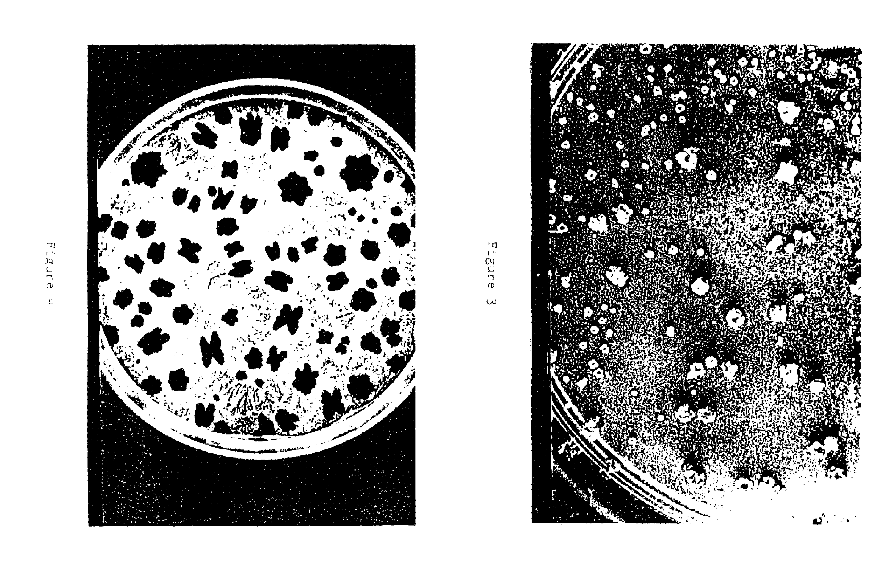

The morphology and size of the colonies as shown in Figures 2-6 will depend on the strain in question, the number of inoculated cells per plate, and essentially, on the composition of the medium and the strategy used to decrease the water activity.

However, there is a common factor for all the colonies obtained : geometrical shapes that look like crystals This aspect was analyzed by scanning microscope and will be described hereinafter.

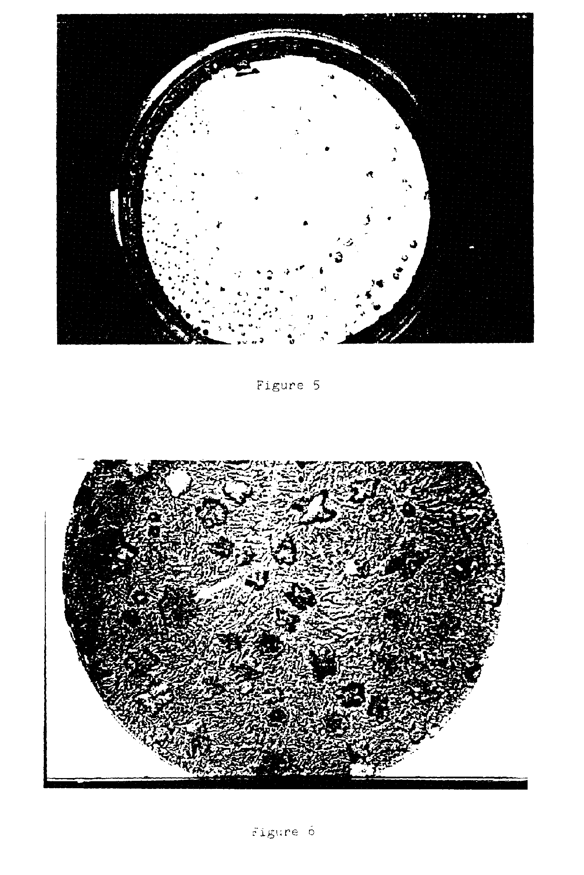

Considering that in agarized media, the loss of water by evaporation is slow, an additional strategy was employed for the quick formation of colonies on a plate. 3 cm³ of salt solution 10X with respect to the salt concentration of the ferrous medium used in previous tests, but without agarizing, were distributed per plate and then the plates were inoculated. The amount of water was less and the evaporation velocity was higher than in the agarized media. As soon as a fine film of salts was deposited on the surface of the plate glass and the typical brightness of excess humidity was lost, colonies developed in a few hours attached to the plate glass as illustrated in Figure 5.

Once again, this form of culture emphasizes the capacity of adhesion and high bacterial multiplication velocity in highly dehydrated environments.

### Microbian movement through surfaces

The above tests revealed that bacteria are able to move throughout the plate even on rather dry agarized media. When working with liquid inocula it was impossible to obtain the direct attachment of the bacteria at the inoculation place even when the medium was highly dehydrated

Microbial movement on humid sulphides and minerals has been also detected, as will be discussed hereinafter.

Microbial movement through an agarized surface, even when it is rather dry, and the requirement of a highly dehydrated agarized media for cell attachment and colony formation, are characteristics of the so-called gliding bacteria. They conslilule a group laxonomically heterogeneous bacleria and are believed to phyLogeneLically arise from different roots

Yet, there are several characteristics that most, or all of them, have in common: The cell wall is typically Gram-negative. In many cases a lipopolysacharide component has been isolated and characterized. Some gliding bacteria are connected with the secretion of a mucilaginous material, causing the cells, in a liquid medium, to group or adhere to the walls of the cultivating container These characteristics are similar to those found in bioleaching bacteria.

The environments and metabolism in which gliding bacteria have evolved favored the development of motility on surfaces. In general they transform substrates which do not diffuse, so that the microorganisms have to roam about in order to find them. It has been demonstrated on synthetic media that gliding is essentially dependant on the humidity and concentration of nutrients.

It should be considered that microorganisms with bioleaching capacity have evolved in mineral environments insoluble substrates are in low concentration and finely disseminated. Only the development of surface spreading mechanisms or surface translocation, has allowed them to evolve.

These characteristics have an enormous potential for technological exploitation. It should be considered that in order to achieve high yields in the bio-oxidation of compounds that are disseminated into mineral ores, at the moment of obtaining the required dehydration conditions to enable a stable bacterial attachment, each particle to be transformed must be in contact with at least one cells. If this is not the case, on subsequently moisturizing and dehydration stages, the cells will be allowed to move and spread to reach new particles.

### Studies of generation times

As previously indicated, the slowness of bioleaching processes in the conditions experimented so far, is essentially due to the low bacterial multiplication velocity.

The development of bacterial colonies in a few hour's time, clearly indicates that when the bacteria is attached to a solid substrate with low water activity, they multiply quickly

The generation times of the ATCC 19.859, BA₁ and BA2 strains were determined in order to compare them in two systems. One was a conventional ferrous liquid medium shaken at 30° C. The development was followed up by extracting daily samples and the number of cells was determined by dilution and count in plate.

During the exponential phase corresponding to the highest multiplication velocity the minimum generation times were determined and they are indicated in Table III as generation times corresponding to free growth in a liquid medium.

The other system corresponds to the development in a medium of the same composition as the above, but solidified with 0.35% of agarose and highly dehydrated The mean generation times were determined considering that each colony originates in one cell, and taking as a developing time a period starting half an hour before the first evidence of growth was detected by stereomicroscopy until the moment when there were clearly evident colonies which were completely isolated with a toothpick Each colony was suspended in a measured volume of a solution, vortexing in order to liberate the cells. The number of cells in each colony was determined by recount in plate, and considered as an average of three different colonies.

Table III indicates the mean generation times when the strains grow attached to a highly dehydrated solid medium.

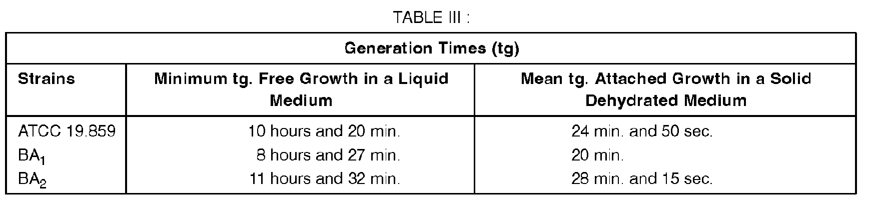

These results demonstrate that optimum microbial development corresponds to low water activity conditions or to a rather dry environment.

### Studies of the relation solid product-bacteria by scanning-microscopy

As previously mentioned, the colonies of the tested strains have in common angular forms and crystal-like appearance, although a dense bacterial population is present when they are suspended in a liquid medium and undergoing microscopic observations. As expected, the oxidized products from their metabolism, at so low water activity conditions are in solid state.

In order to examine the distribution and the relationship bacteria-solid product of such colonies, microscopic scanning observations were carried out.

As an example, the observations made on star-shaped bacterial colonies of approximately one centimeter in diameter, such as the one indicated with an arrow in Figure 6, will be described. These colonies were obtained from a ferrous agarized medium in which the water content was reduced according to the above described strategies

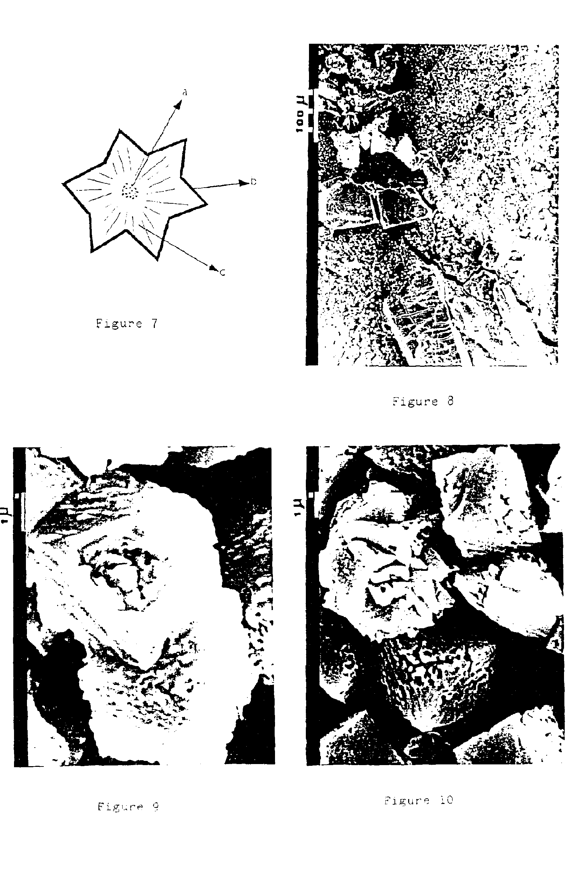

Figure 7 is a schematic drawing of a standard colony in which differentiable characteristic zones of the colony are indicated a) centre b) border c) intermediate zone between centre and border.

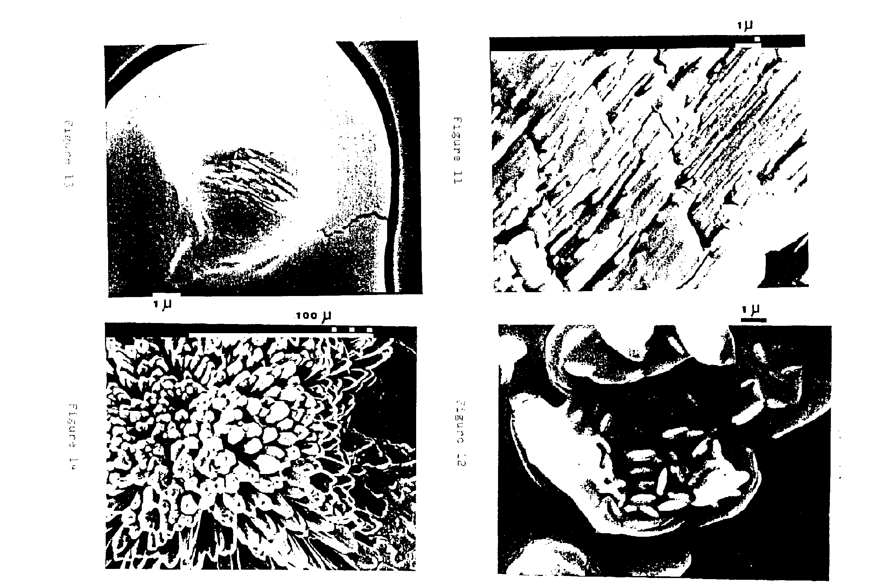

The centres of the colonies such as the one indicated with a), exhibit, under direct observation, a granular appearance or degradation The corresponding scanning photographs show minor units of disintegrated cubic form, as shown in Figure 8, with their walls severely perforated as shown in Figures 9 and 10. This suggests that the bacteria remain temporarily occluded in the solid product they form, and afterwards, they perforate the solid product and abandon it.

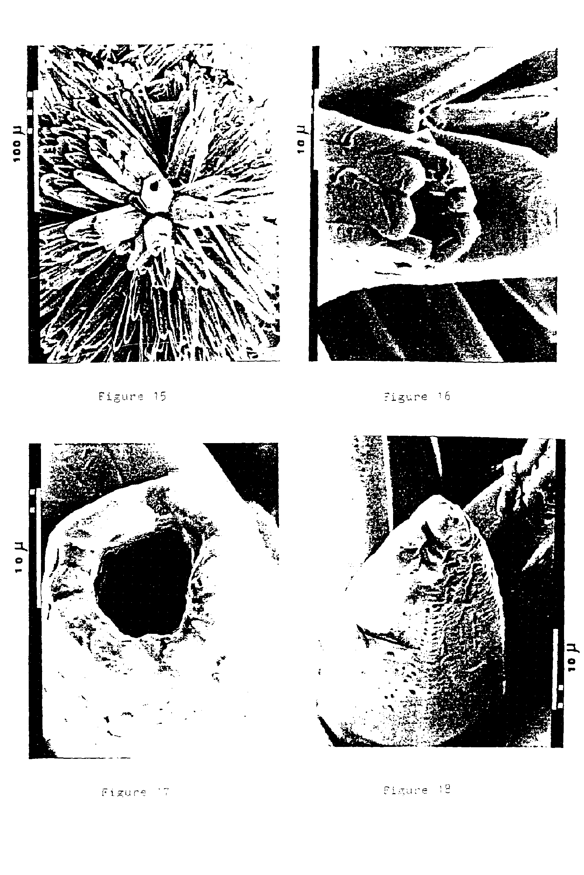

When the surface of the zone between the centre and the border, as represented by c) in Figure 7, was examined by scanning, only a solid compound, looking like crystals ordered parallel to the plane of the colony was observed, as shown in Figure 11. However, if a colony is washed with a slightly acid solution prior to fixing for analysis by scanning, bacteria in the inside can be observed without an organized distribution, as shown in Figure 12. However, if the crystal-like solid from this zone is destroyed mechanically with a sterile toothpick prior to observation by scanning, the bacteria appears distributed in planes and in ordered directions, as shown in Figure 13.

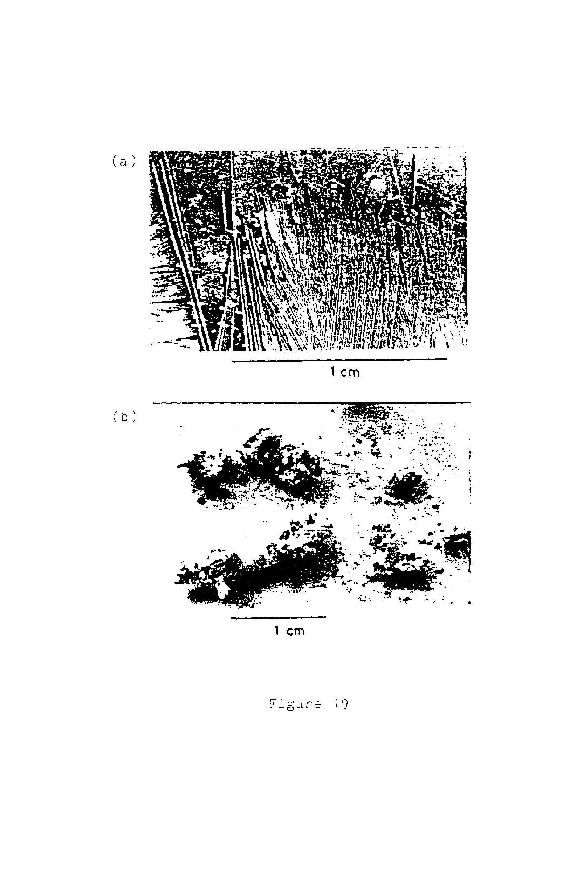

When the border of the colony indicated with b) in Figure 7 is analyzed by scanning without introducing any previous alteration, crystal-like bodies are observed, but these differ from those of zone c) because they stand up from the plane of the colony and groups of them adopt the form of a cluster, as shown in Figures 14 and 15. Although all the bodies from the border have a similar form, differences among them can be seen basically at their upper extremity which is more exposed to air and remote from the base. Some of these bodies have their upper extremities closed, but others are openended, as shown in Figures 16 and 17. Some of the bodies show fractures on the upper as shown in Figure 18.

Observations carried out in the border of the colonies, together with the fact that 24 to 48 hours after the analyzed colonies were obtained, minor colonies began to form in the surroundings of the original ones without any evidence of communication through the substrate, permit associating the phenomenon to a kind of bacterial surface translocalion known in microbiology as "darting". It is produced by the expansive forces developed in an aggregate of cells inside a common capsule and results in the ejection of cells from the aggregate.

### Bacterial development in relation to the nitrogen source

Although bioleaching bacteria are extremely efficient in scavenging nitrogen in the form of ammonia, the scarcity of nitrogen in leach liquors may limit the efficiency of bacterial leaching operations. On the other hand the addition of ammonia to leaching solutions, implies an additional cost.

The ability to fix nitrogen is important for any organism inhabiting environments deprived of nitrogen.

It has been demonstrated that, at least T. ferrooxidans is capable of fixing atmospheric nitrogen in limited conditions of oxygen supply and has been characterized as having a nitrogenase system, and nif genes from this system have been cloned

However, in situ studies have not demonstrated nitrogen fixing activity in heap leaching operations. Although the physiological conditions under which nitrogen is fixed vary, the nitrogenase enzyme is conserved and is usually sensitive to oxygen. The nif proteins are oxygen-labil and the system responsible for nitrogen fixation has been found to function only when protected from oxygen.

The physiology of an obligate aerobe microorganism, of free life which possesses a nitrogenase system has stated, in the scientific field an apparently biological contradiction, that until now, had not been solved. Nevertheless, it was expected, that if bacteria had evolved with a nitrogenase system, conditions in which this system functions efficiently must exist.

The growth of microorganisms with bio-oxidation capacity indicated in Table II, using synthetic siilphides as substrate, under the conditions mentioned below (which are characterized by low water activity) and which result in the corresponding oxidized solid, crystal-like products containing the bacteria, is totally independent from the addition of nitrogen.

As corroboration of the nitrogen source and the elements required for microbial growth, although in small quantities (such as phosphorus, potassium, magnesium), the bacterial development was analyzed in relation to the medium composition, using analytic grade ferrous sulfate as energetic substrate.

In order to introduce in the analysis system the least possible quantity of components, and considering that gelling agents may bring impurities the method of colonies formation in the salts deposited on the plate glass was used.

Plates, nine centimeters in diameter, were prepared distributing either of the following media on each plate:
a) 3 ml of an analytic grade S0₄Fe.7H₂0 10% solution, adjusted to pH=1.8
b) 1,5 ml of an analytic grade S0₄Fe.7H₂0 20% solution adjusted to pH=1.8, plus 1.5 ml of a solution adjusted to pH=1.8 containing: KCL, 0.1 g; K₂HP0₄. 0.25g; MgS0₄. 7H₂0, 0.25 g Ca(NO₃)₂ 0.01 g; H₂O, 800 ml.

Different strains gave equivalent results. In the plates containing only analytic grade ferrous sulfate, there was no colonies formation. In some cases, the typical slime tracks of a kind of bacterial surface translocation called "social gliding" remained engraved, as shown in Figure 19 (a). It is known that this phenomenon is induced by nutrient defficiency.

In the plates containing, besides ferrous sulphate, small quantities of salts which provide life-supporting elements, although without the addition of a nitrogen source, colonies of approximately mm developed, as shown in Figure 19 (b).

This suggests that in high dehydrated conditions, the nitrogenase system is efficient for fixing atmospheric nitrogen. We could surmise that the solid product in which the bacteria remain occluded, at least temporarily protects the nitrogenase system for oxygen.

Characterization of these bacteria as free life microorganisms must be scientifically discussed and studied in depth.

### Microbial Development and Bio-Oxidation of Sulphide Minerals

The principles described previously with respect to bioleaching bacteria in relation to water, were applied to bio-oxidation of synthetic sulphides, natural specimens, concentrates and ores.

A weighted and sterile quantity of each concerned substrate was placed on Petri plates and homogeneously distributed on all the surface. Afterwards, the substrate was moisturized with a sulphuric acid solution, the most convenient volume and concentration of which were determined for each particular case, taking into account the following criteria :

- The most convenient volume per mass unit of the substrate under consideration is the minimum volume that ensures the total and homogeneous acidification of the substrate.

This volume will depend on the physical and chemical characteristics of each particular substrate and in the case of porous minerals acidification through the pores must be ensured. Nevertheless, the volume must be as small as possible in order to decrease the losses of time inherent to the subsequent dehydration of the substrate.

- The most convenient concentration of acid is the amount of acid which in the most convenient volume, ensures neutralization of the mineral, prevents compacting, provides the amount of acid for an efficient bacterial development, and contemplates possible losses of acid by evaporation.

Criteria established in respect of the optimum pH for microbial growth in liquid media, are not valid for the development conditions here proposed for several reasons. The acidification volume is many times smaller than the volumes of acid solutions provided in liquid systems If the acid concentrations were the same or equivalent, the availability of acid would be very low in this new system. The metabolic conditions and probably the cellular surface composition of the bacteria in such diverse environments, are different Also, when the system reaches the high dehydration degree required for quick bacteria development, the pH concept is not applicable.

It should be mentioned that the existing impurities in natural specimens, and even in the synthetic sulphides tested, were generally enough to provide the required small quantities of elements essential for bacterial growth, such as phosphorus, potassium, magnesium, etc. Nevertheless, this must be analyzed for each substrate.

The plates were inoculated with any of the strains indicated in Table II and subsequently incubated in such a way as to facilitate a quick loss by evaporation of water introduced during the acidification. In all cases, when the substrate acquired a dry appearance, the microbial development associated with the corresponding bio-oxidated solid product, was obtained in a few hours.

Tests using different substrates, which are not limitative, will now be described.

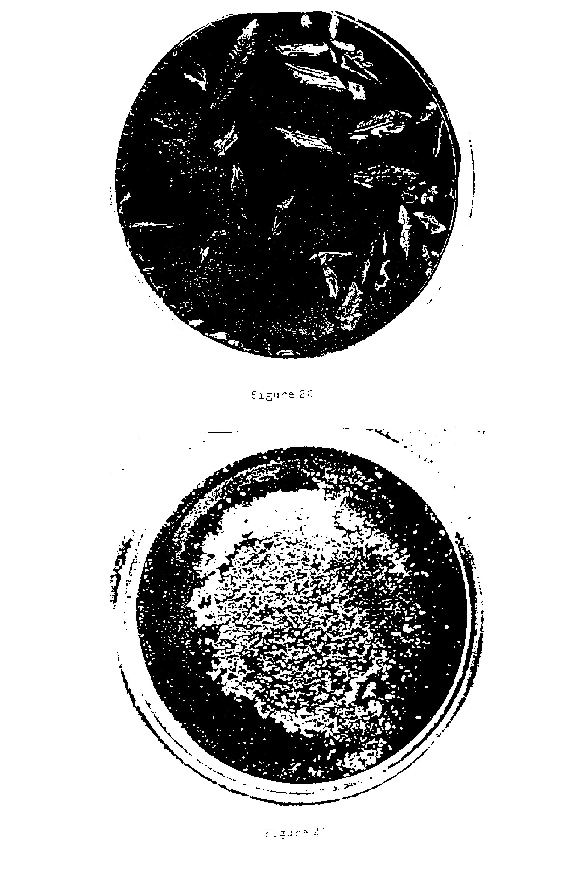

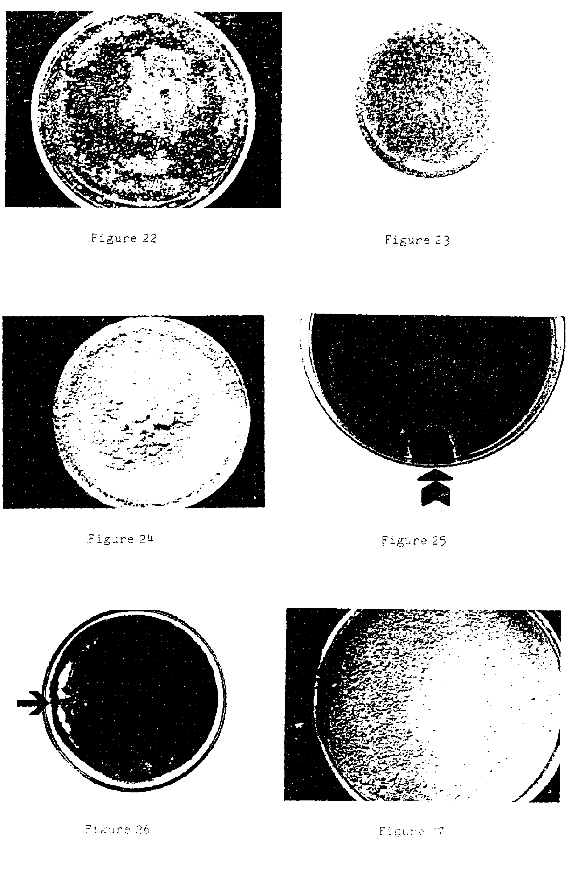

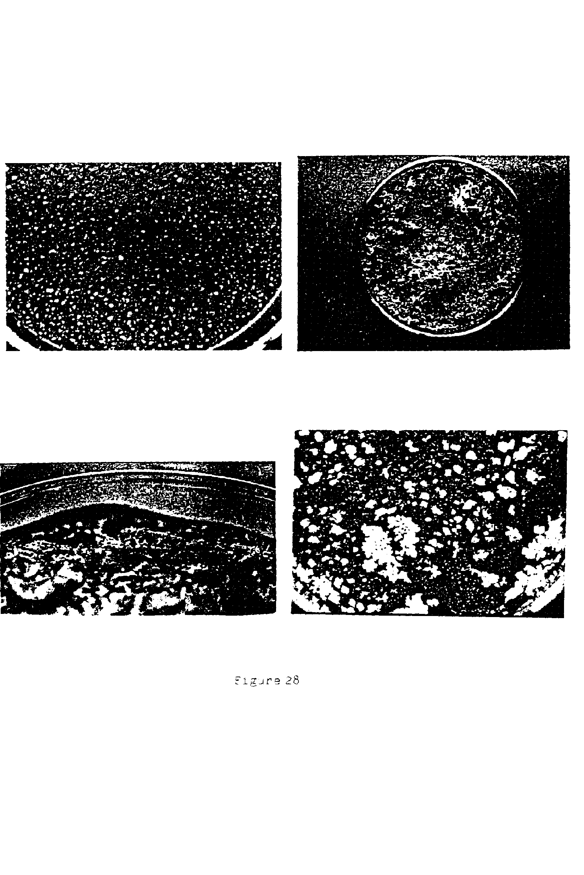

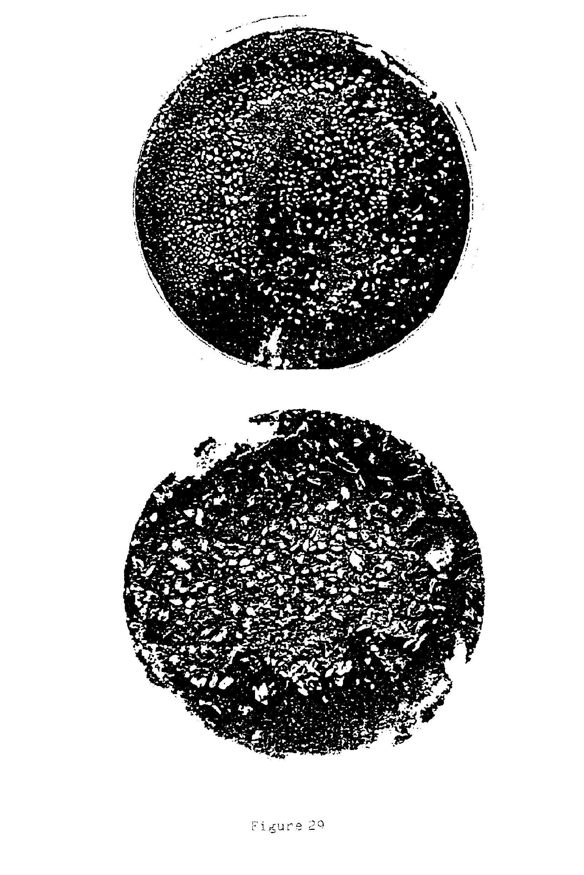

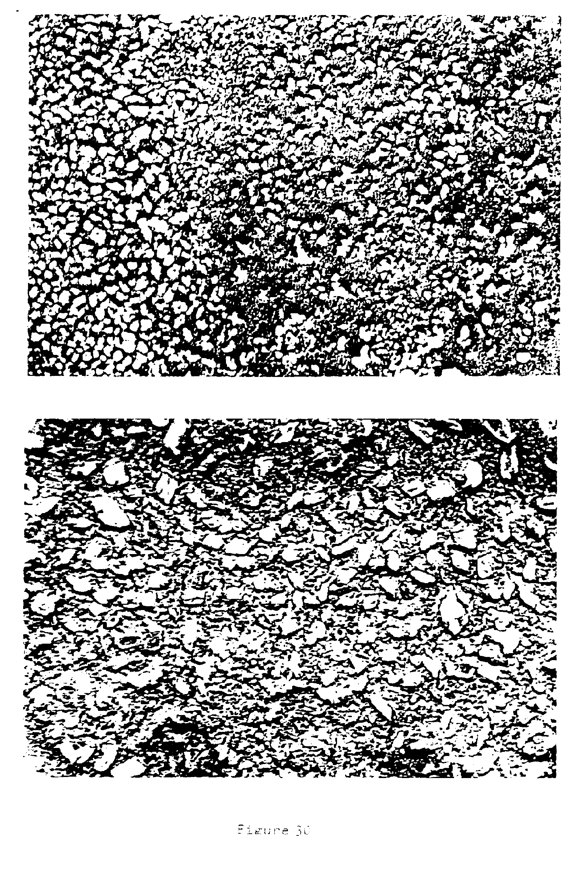

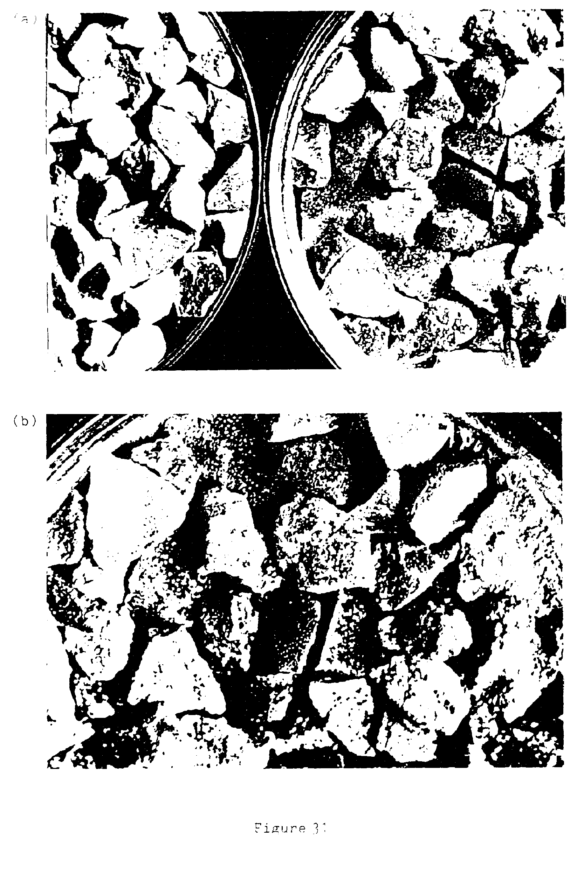

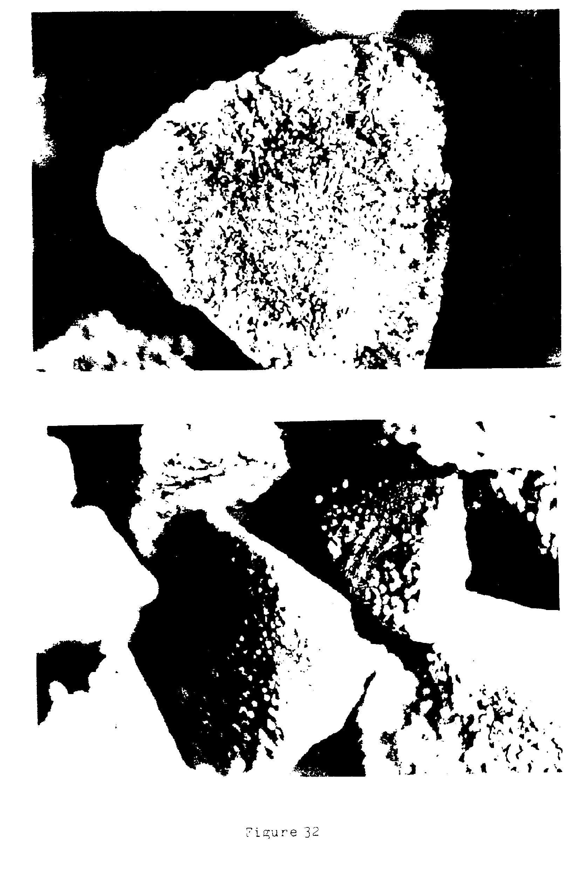

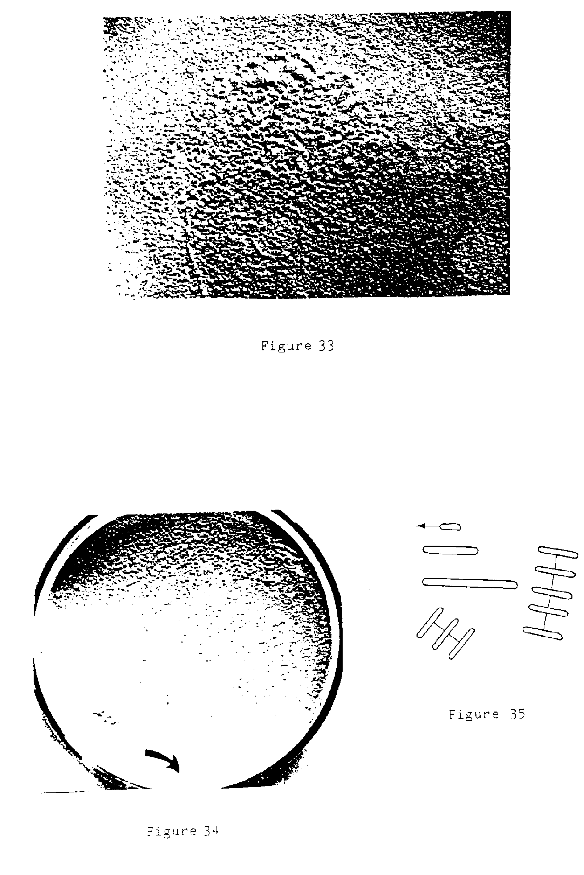

a) A thin layer of dry and sterile synthetic copper sulphide was distributed in a Petri plate, 9 cm in diameter. Next, it was acidified adding, drop by drop, 1 5 cm³ of a sterile 0.1 N solution of sulphuric acid. It was inoculated in the centre of the plate with 10 microliters of an inoculum of the BA2 strain prepared by dissolution of a copper sulphate crystal from a previous culture, in a 0.06 N solution of sulphuric acid. The inoculum was prepared at the time of inoculating the newplate, so as to keep the bacteria in a liquid medium during the least possible time. It was incubated at 30°C. When the substrate acquired a dry appearance, the first evidences of development were observed, and a few hours later, blue crystals of copper sulphate were obtained. They were 0.5 cm wide by 1.5 to 2.5 cm long, as shown in Figure 20. These crystals, are themselves, bacterial colonies.
b) A plate prepared as in a. was inoculated at the centre with 20 microlites of a dense inoculum of the BA₃ strain and was cullivaled at 37° C keeping the plale halfway open in order lo facilitate the loss of wafer by evaporation. After twelve hours, a development as illustrated in Figure 21, was obtained. It proves the bacterial movement capacity while humidity was present.
c) A plate prepared as before was acidified by adding 2 cm³ of a 0.06 N solution of sulphuric acid It was inoculated with 10 microliters of an inoculum of the CRT strain prepared as previously described and incubated at 85°C. After six hours, the first evidences of development were detected. Two hours later a development as the one shown in Figure 22 was obtained
d) A natural pyrite specimen (FeS₂) of high degree of purity, crushed to -0.15 mm (-100 mesh) when dehydrated after acidification, was used as a substrate. It showed a high tendency for compacting impeding bacterial development. It was determined that by using a 1:1 ratio between the weight of pyrite and the acidification volume, the most convenient concentration of acid is 0.45 N. Thus, placing 0.5 g of sterile pyrite on a plastic plate, 5.5 cm in diameter, and adding 0.5 ml of a sterile 0.45 N solution of sulphuric acid, and by rotating movements, a fine homogenous layer of acidified substrate was obtained. The centre of the plate was inoculated with an inoculum of the CM₁ strain adapted for growing in pyrite by previous cultures. Keeping the plate closed, it was incubated at 30°C. In these conditions, twenty four hours were required for the substrate to acquire a dry appearance. At this time, the first evidences of development were observed and ten hours later, a development as shown in Figure 23 was obtained.
e) A natural galena specimen (PbS) of high degree of purity, crushed to -0.37 mm (-40 mesh) was used as a substrate 1 g of sterile galena was placed on a plastic plate of 5.5 cm in diameter. It was acidified with 1 ml of a sterile 0.24 N solution of sulphuric acid and it was inoculated with the CM₂ strain from a previous culture in CuS. It was incubated at 37°C. Between 18 and 22 hours later the beginning of growth was detected. Six hours later, glassy white bodies with a crystalline appearance were obtained The size was the same as the galena grain, but contrasting with the greyish tone of the latter It was determined, by microscopic observation, that they carry a dense bacterial population.
f) 2 g of the same substrate as in e., were placed in a glass plate. 2 ml of a 3.0 N solution of sulphuric acid were added. It was inoculated with a CRT strain. It was incubated at 90°C. After six hours, the first evidences of development were observed. Four hours later, white solid bodies of similar characteristics as those described in e , were obtained.
g) A concentrate of sphalerite (ZnS) containing 56% of zinc was used as substrate. 2 g were placed on a plastic plate 9 cm in diameter. 1.5 ml of a sterile 0 24 N solution of sulphuric acid was added By rotating movements, the acidified substrate was distributed throughout the plate. The centre of the plate was inoculated with the ATCC 19.859 strain, previously adapted to growth in ZnS, and suspended in S0₄H₂ 0,06 N at the moment of inoculation. It was incubated at 30°C, keeping the plate closed. About 48 hours later, the first evidences of developmente were observed, and twelve hours afterwards the development shown in Figure 24 was obtained, associated with the typical white colour of zinc sulphate.
h) A plate prepared in the same way as in g) but without inoculation was incubated at 30°C. until 90% of the water during acidification was lost as determined by loss of weight. After that, it was inoculated with a liquid inoculum, in the place indicated by the arrow in Figure 25. Twelve hours later the typically white development was obtained in the surrounding area of the inoculation place. No development was observed in the inoculation place, which had a higher degree of humidity due to the inoculum. This indicates that the bacterium moves from the most humid area to the less humid area where it is attached transforming the substrate.
i) A glass plate carrying 2 g of the same concentrate of sphalerite (ZnS) as the one used in g) and h) was acidified with 2 ml of a sterile 0.18 N solution of sulphuric acid It was inoculated with the CRT strain from a previous culture in ZnS, but instead of suspending the inoculum in a solution. The inoculation was carried out by direct transfer, with a thick needle, of solid zinc sulphate to a marked edge of the plate indicated with an arrow in Figure 26. It was incubated at 96°C. with the plate half open to enable the quick loss of humidity After two hours the first signs of development were detected and two hours, later a development associated to the typical white colour of zinc sulphate, was obtained in the inoculation area, as shown in Figure 26.
j) A natural antimonite (Sb₂S₃) specimen of high purity degree, crushed to -0.15 mm (-100 mesh) was used as substrate. Of the substrates tested, this one was the hardest to acidify because of its hydrophobicity. Obtaining a homogenous acidified pulp, distributed on the surface of the plates required higher volumes of acid solution than in previous cases. Besides, in order lo oblain a homogenous pulp, a slerile spaLula should be used when mixing on the plate. This system also had a high tendency to compact, which obstructs bacteria development. This explains the need to use higher concentrations of acid than in previous cases. A wide variety of tests were carried out to determine the most convenient volume and acid concentration. For any of the strains indicated in Table II, the best results were obtained by homogenouslydistributing 0.5 g of Sb₂ S₃ and 1.5 ml of an acid solution 0.6 N in polystyrene plates, 5.5 cm in diameter. It was incubated at 30°C keeping the plates open to enable the loss of water by evaporation Between 3 and 4 days later microbian development took place associated with the deliquescent white bio-oxidation product in solid state, as shown in Figure 27. It is possible to reduce the volumen of the acidification solution by adding tensoactive agents, as such as sarcosyl (1 %), which further more enhance the microbial development. In this manner, it has been possible to obtain development in 24 to 30 hours
k) Plates, 9 cm in diameter, were prepared with a thin layer of dry, sterile synthetic cobalt sulphide (CoS). They were acidified adding drop by drop, 0,5 ml of a sterile sulphuric acid solution 0,3 N, so as to acidify homogeneously all the substrate. Plates were inoculated with different strains from previous culture in cobalt sulphide and were incubated at 30°C. Twenty four hours later, the developments shown in Figure 28 were obtained. The developments presented the typical pink to red color of cobalt sulphate. The bio-oxidized product obtained in solid state may be separated by screening
I) A copper concentrate was used as substrate. The mineral composition considering the base as 100% copper sulphide minerals, indicates that the predominant specimens are chalcosite (64,29%) and enargite (21,96%). 1 g was placed on each plate, 9 cm in diameter. It was acidified with 1 ml of a sulphuric acid solution 0,3 N. The plates were inoculated with the BA₁ and CM₁ strains from previous cultures in the same concentrate. It was incubated at 30°C, keeping the plates open. Twenty four hours later, developments as shown in Figure 29 were obtained. Figure 30 shows photographs of the same developments, but taken at a shorter distance.
m) A mineral ore from the Argentine deposit of "Campana Mahuida" comprising chalcocite (Cu₂S) as the predominant copper specimen, triturated to 0.64 cm (1/4 inch), was tested. 30 g of the mineral ore were placed in a plate, 9 cm in diameter It was acidified adding 20 ml of sulphuric acid solution 0.45 N. It was inoculated with the CM₁ strain from a culture in synthetic copper sulphide It was incubated at 37°C keeping the plate open so as to facilitate quick dehydration After twelve hours, when the mineral ore has acquired a dry appearance, the growth began. Four hours later, over the dry stones, the bacterial development associated with the oxidation of the sulphide was obtained. Figure 31 (a) shows the mineral ore non subjected and subjected (left and right respectively) to bio-oxidation. Figure 31 (b) shows the bio-oxidated mineral. Figure 32 shows photographs taken at a shorter distance, of the colonized and bio-oxidated mineral ore.
n) A copper mineral crushed to -0.15 mm (-100 mesh) comprising 2.1% of chalcocite (CU2S) was used as substrate. 5 g of sterile mineral were placed in polystyrene plates of 8.5 cm in diameter. It was acidified with 5 ml of sterile 0.4 N solution of sulphuric acid, and by rotating movements the pulp was homogenously distributed throughout the plate. 100 microliters of an inoculum of the CM₂ strain from a culture in CuS were distributed by drops and the inoculated substrate was incubated at 37°C. After 48 hours, and when the substrate had a dry appearance, the development started to appear looking like elevations in the more dehydrated edges of the plate. Twelve hours later a development as shown in Figure 33 was obtained, it was characterized by irregularities of the mineral layer associated to blue little crystals.
o.- A plate of the same composition as the one indicated in (n) was used but it was incubated and prepared on a slightly inclined plane, so that the pulp is thinner at one edge than at the diametrically opposite edge The pulp was inoculated at the thicker edge indicated with an arrow in Figure 34. It was inoculated with 20 microliters of the same inocula mentioned in (n). It was incubated at 37°C. As expected, the thin edge was dehydrated first. After twenty six hours the first evidence of development and microbial transformation on the thin edge was detected and six hours later, the development shown in Figure 34 was obtained.

Several days later, when the plate was completely dehydrated with respect to the humidity added in the acidification, there was no evidence of development in the rest of the plate. This indicates that while there is excess of humidity, the bacteria move through the substrate, curiously in a path and associated to a negative humidity grade, producing a stable attachment on the area which is dehydrated first.

### Most Relevant Conclusions Regarding the Physiology of Bioleaching Microorganisms

Knowledge of the physiology of these microorganisms permils solving specific problems of oplimizalion of the biological oxidation processes with a view to intensify them.

Although substrate oxidation activity by different bacterial species, and even by different strains of the same species, is variable and determined by the prehistory of their existence, the previous mechanisms which must be fulfilled for the efficient biological oxidation, such as a stable substrate-cell attachment and the destruction of the sulphide mineral lattice, are governed by similar conditions.

From the previously described tests is follows that
1) These bacteria have evolved in mineral environments of low water content or at least that are not immersed in a liquid system. The water contained in the minerals and the humidity of the environment is enough for their development.
2) Biological oxidation of a solid insoluble substrate implies an interaction substrate-cell through an adhesion mechanism. A stable adhesion permits the quick transformation of the substrate with a consequently high, bacterial multiplication velocity. Excess of water prevents or makes difficult such adhesion.
3) If there are enzymatic systems involved on the destruction of the sulphide mineral lattice, such enzymes should not be diluted or washed out from the reacting surface.
4) In conditions of low water activity, the microbial metabolism is activated, decreasing considerably the generation times with a consequently high microbial multiplication velocity associated to a high substrate oxidation velocity
5) Under such conditions the microorganisms multiply, remaining occluded, at least temporarily, into the solid, crystal-like bio-oxidation product.
6) At least the tested microbial strains, when grown in a high dehydrated media, do not require the addition of a nitrogen source, suggesting an efficient fixation of atmospheric nitrogen.
7) Most of the microorganisms have mechanisms for dealing with a degree of water stress, but relatively few have evolved with physiological adaptations that enable them to grow well in low water activity (a_{w}) environments Many terms have been used to describe these microorganisms halophilic, osmophilic, osmotolerant, xerophytic, xerophilic, etc. Of these terms, xerophilic (from the greek =dry-loving) is perhaps the most appropiate for describing the microorganisms here tested.

The fact that these bacteria have not been analyzed in such a frame of conditions is attributed to the procedure of isolating them starting from the acid drainage of the mines, under the preconception that there are only ways of life on systems with abundance of water, without considering that these bacteria evolved transforming minerals which usually are not found on nature, suspended on a watery environment and although minerals may look dry or dehydrated contain a percentage of water, at least what is at equilibrium with the humidity of the environment.

In our experience samples of minerals that are apparently dehydrated, have a rich microbial flora, specially on the surfaces exposed to the air and they are viable cells.

Considering to the basically antagonistic relation-ship in respect to water between the traditional processes included in Biohydrometallurgy and the microbial bio-oxidation conditions here described, one may propose the term "Biodehy- drometallurgy".

### Benefits derived from the Technological Application of These Principles

The benefits of the application of the principles of the present invention will be obvious, if the following is considered:

- Under any of the systems until now developed heretofore in Biohydrometallurgy: tank leaching, in-situ, waste dumps and heaps, the microbial flora is compelled to develop in an aqueous environment. In such conditions, the microbial multiplication velocity and the substrate bio-oxidation velocity are limited. These entail long processing times, in the order of several months, which create a technical and economical barrier. By application of the principles hereby described, it will be possible to obtain equivalent extracting yields in terms of days. Considering that the microorganisms develop and transform their substrates in terms of hours, the determining parameters of the total processing time is the dehydration velocity in each system and the strategy employed in the acidification and the proper inoculation, incorporating to the system the least possible amount of water

Considerably shorter processing limes imply lhal for an equivalent production, much smaller processing volumes would be required, with the consequent economy in capital investment.

The management of smaller processing volumes opens the possibility of operating precisely controlled systems.

Traditional systems have an important energetic cost In tanks or reactors, pulp must be continuously shaken and aired. On other systems, the mineral is subjected to percolation of the acid leaching solutions with the attendant energetic and capital costs. These costs become very important for the long processing periods required. Fast processes will imply less operational costs

### The most convenient form to practice the invention

The most convenient volume and acid concentration must be determined for each system according to the above mentioned criteria, in order to neutralize the mineral ore or the concentrate, prevent their compactation and provide the adequate environment for the microbial development.

Once the substrate is acidified and inoculated, the loss of the water excess must take place either by spontaneous evaporation, induced by a dehumidifier, or by flow of air through the mineral ore or the concentrate.

The substrates to be oxidized via microbial action are found in mineral ores, generally disseminated in small particles. The transformation of each particle requires the attachment of at least one cell. Particles that at the time they reach the convenient dehydrating level meet the above condition, will be transformed in a few hours turning into a soluble solid product, which at the same time constitutes a microbial colony If at the following stage, the mineral ore is moisturized to dissolve at least partially, the already formed solid product, the bacteria will be released and under humid conditions may glide or reach by any other means new particles of substrate.

The previous operations may be repeated as many times as required in order to obtain the expected extraction yield and this will depend on the characteristics of each particular system, whether the substrate is a mineral ore or a concentrate.

Finally the bio-oxidated solid products must be separated by washing or screening, when a concentrate is subjected to bio-oxidation. If solubilization is used, the pH of the washing solutions will depend of the solubility of the oxidized compounds involved.

Considering that the solubility of metal can also take place indirectly, that is the ferric iron resulting from microbial oxidation, can react chemically with sulphides oxidizing them to soluble forms, the convenience of maintaining for a certain time the washing solution in contact with the mineral for a certain time, in order to permit eventual increases of the extraction yields by the indirect way, must be analyzed for each particular case.

In the case of metals bioleaching processes, the washing solutions will carry the products of interest, whereas the solids will constitute the residuum. In the case of microbial purification processes, for example the desulphuration of coal or the purification of precious metals, the washing solutions will carry the residuum, while the solids will constitute the product of interest.

### EXAMPLES

The performance of this invention is demonstrated with the following examples, which are not limitative :
Examples I and II ilustrate the quantitave differences of biological activity of the oxidation of metallic compound in liquid media and in conditions of low water contents.
Example III explains the application of the invention to a mineral ore

### EXAMPLE

A natural pyrite specimen (FeS₂), with a high degree of purity containing 43.5% Fe; 49.67% sulphur and 6.83% impurity, crushed to -100 mesh and sterilized in three consecutive days by flowing steam, was used as substrate.

An inoculum corresponding to the CM₁ strain previously adapted to growth in pyrite was used. In all cases, the corresponding sterile controls were carried out simultaneously.

The biological oxidation was tested in a conventional liquid system, with or without the addition of ammonia, and in a system of low water content in accordance with the principles of the present invention.

The biological activity was determined by measuring of soluble iron by absorption spectrophotometry The number of cells was determined by recount in plate in agarized ferrous medium.

In the liquid medium, test were carried out in 300 ml erlenmeyers containing 5 g of pyrite, 95 ml of sulphuric acid solulion, wilh or without the addition of 0.3 g of ammonia sulphale and adjusted to pH=1.7. IL was inoculated with 5ml of a culture of the CM₁ strain containing 2x10⁸ cells/ml. In sterile controls, 100ml of solution was added instead of 95 ml. The erlenmeyers were incubated in a shaker at 30°C.

Iron solubilization kinetics was followed by periodic determinations of soluble iron concentration in aliquots taken from the leaching solution.

The rate of iron extraction was estimated from the linear part of a plot representing the total biologically disolved iron as a function of time and refering this value to each inoculated cell in the system.

The rate of iron solubilization was expressed as solubilized iron milligrams per hour and per inoculated cell. In the test with ammonia nitrogen, this value was 1.68 x 10-⁹ mg/h cell. In the test without ammonia nitrogen there was basically no difference with the sterile control

For the test in dehydrated solid medium, the most convenient volume and acid concentration were previously determined. The best volume was the one corresponding to a ratio of 1:1 pyrite weight in grams to acid solution volume in mililiters. The most convenient acid concentration was 0.45 N.

In order to follow up the kinetics for soluble iron, polystyrene plates, 5.5 cm in diameter were used, each containing 0.5 g of pyrite and 0.5 microliters of a sulphuric acid solution 0.45 N By rotational movements a fine film was spread all over the surface. Each plate was inoculated with 360 cells of the CM₁ strain contained in 20 microliters of a sulphuric solution 0.06 N. The number of cells was determined by count of colonies in plate in an agarized ferrous medium and coincided, with a mistake of ± 7% with the colonies which can be counted in pyrite plates These plates after reaching the highest development were of a similar appearance to the one shown in Figure 23. 20 microliters of a sterile sulphuric acid solution 0.06 N were added to the corresponding sterile controls . All the plates were incubated at 30°C.

Periodically, one sterile and one inoculated plate were subjected to soluble iron determination by absorption spectrophotometry. Solubilized iron by biological oxidation was plotted as a function of time.

After twenty two hours, when the plates had acquired a dry appearance, biological oxidation was initiated. Starling from twenty six hour and for a period of eight hours, the highest biological oxidation rate was obtained, which was of 8.79 x 10-⁴ mg/h cel.

Comparising this value with the one obtained from the liquid medium with ammonia, results a difference of five orders of magnitude.

### EXAMPLE II

Synthetic CuS was used as substrate It was inoculated with the BA₁ strain

Just like in example I, the biological oxidation in conventional liquid medium was carried out with and without ammonia aggregate, and in a dehydrated solid medium according to the criteria already described. In all cases, corresponding sterile controls were conducted simultaneosly. Soluble copper was determined by absorption spectrophotometry. The biological oxidation was determined in each case as the difference of solubilized copper between the inoculated system and the corresponding sterile control.

The liquid medium leaching was carried out in erlenmeyers containing 5 g of CuS and 95 ml of sulphuric acid solution, with and without the addition of 0,3 g of ammonia sulphate. The pH of the solution was adjusted to 2. It was inoculated with 5 ml of an active culture of the BA₁ strain, containing 2 x 10⁸ cel/ml. In sterile controls, 100 ml of acid solution were added instead of 95. The erlenmeyers were incubated in a shaker at 30°C.

The copper solubilization kinetics was followed up by periodical determinations of soluble copper in aliquots taken from the leaching solution. The rate of copper biological solubilization corresponding to the lineal part of a plot representing the biologically solubilized copper, as a function of time was determined. It was expressed in milligrams of solubilized copper per hour and per inoculated cell. In the lest with ammonia nitrogen this value was 5.1x10⁻⁹ mgr/hr cel. In the test without ammonia nitrogen there was basically no difference with the sterile control.

For the test in dehydrated solid medium, plates, 9 cm in diameter, were prepared by adding to each one 2 g of CuS and 2 ml of H₂0. A pulp was formed and by rotational movements it was homogenously distributed all over the surface of the plate. The plates were dried in a laminar flux hood untill attaining constant weight. To each plate, 0,5 ml of a sterile 0.3 N solution of sulphuric acid was added distributing it drop by drop so as to acidify homogenously Each plate was inoculated with approximately 40 cells of the BA₁ strain, contained in 20 microliters of a 0.06 N solution of sulphuric acid. The number of cells contained in the inoculum (2,000 cel/ml) was by count of colonies in ferrous agarized medium and it coincided, with a mistake ± 8% with the number of colonies that were obtained in the plates with CuS at the end of development.

To the sterile controls, 20 microliters of a sterile acid 0,06 N solution were added. All the plates were incubated at 30°C.

Soluble copper was analyzed periodically from a sterile plate and from an inoculated plate. After 18 hours when the plates had a dry appearance, the biological oxidation was initiated and continued with an almost constant velocity during eight hours. At the end of this stage the maximum development in terms of the size of the colonies constituted by solid crystal-like copper sulphale, was achieved.

The biological oxidation rate during this period expressed as solubilized copper per hour and per inoculated cell was 0.319 mq/h cel. This value should be compared with the corresponding one obtained from a liquid medium.

NOTE : When a bacterial colony constituted by a solid crystal-like product is suspended in a solution, even when subjected to vortexing and is then examinated with a microscope, it is possible to observe forms like the ones shown in the schematic drawing of Figure 35: Small mobile cells, intermediate and very long non-mobile cells, and groups of cells that appear liked to each other by a very thin filament As a consequence, that at the time of determining the number of cells present in an inoculum by count of colonies in plate, it is uncertain whether each colony originates from a cell or from a group of cells. Nevertheless, considering that the applied inocula in the corresponding liquid and solid medium have the same origin and have been treated in the same way, the biological activity values referred to inoculate cells are valid for comparison purposes, although they should not be considered as absolute values.

### EXAMPLE III

The bio-oxidation of a copper mineral ore was tested, in order to determine the copper extraction yield and the necessary time to achieve it by application of the principles of the invention.

### Characterization of the Mineral Ore

### a. - Chemical Characterization

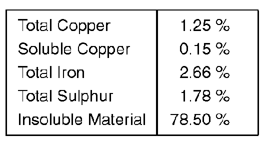

### b. Mineralogic Characterization

The main species resulting from mineralogic analysis are listed below. The percentage of each one is expressed with reference to 100% mineral ore.

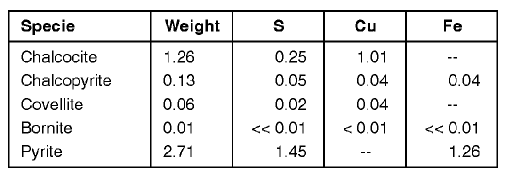

It will be seen that the predominant copper specimen is chalcocite; chalcopyrite, covellite and bornite follow in order of importance.

### c. - Granulometric Analysis

Triturated mineral to 0.64 cm (1/4 inch) was subjected to granulometric analysis using the 4 75 mm (#4) 2.36 mm (#8), 1.00 mm (#16), 0.37 mm (#40), 0.21 mm (#65), 0.15 mm (#100), series. The granulometric distribution is indicated below :

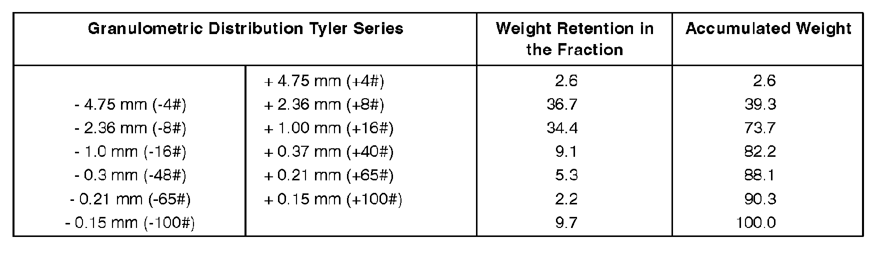

### d.- Natural Humidity Determination

Natural humidity was determined by loss of weight of the mineral at 100°C until constant weight was achieved. Corresponds to 1.5%

### e.- Acid Consumption Test

By a standard test the sulphuric acid consumption of the mineral ore was determined. Such value corresponds to 9.9 g of acid by kilogram of mineral.

### Test: Bio-oxidation on Tray

Two parallel tests were carried out on 35 x 45 cm stainless steel trays Each tray was loaded with 1 kg of mineral and it was treated as indicated below.

The tests took place in a closed room, with a sealed door containing a dehumidifier. Water condensed by the dehumidifier was drained outside with a hose. Thus, the mineral dehydration was facilitated.

The room temperature was maintained between 28 and 32°C during the test.

The mineral, homogenously distributed on each tray was acidified with 800 ml of solution containing the amount of acid determined by the consumption acid test, that is, 9.9 g of sulphuric acid

Each tray was inoculated with 10 ml of an inoculum of the CM₂ strain. The inoculum was prepared by suspension in a 0.06 N solution of sulphuric acid of the white zinc sulphate development, corresponding to the culture of this strain in zinc sulphide. The inoculum was prepared at the moment of inoculation and was distributed homogenously by drops all over the tray. The trays were incubated in the above described conditions.

After twenty six hours, the mineral presented a dry aspect and a strong bacterial development associated to little light blue crystals. Basically it was observed on the surfaces and on the sections of the mineral more exposed to air. As shown previously, the microorganisms move towards the areas which are more quickly dehydrated

At the end of this stage, and at the end of each of the following ones, samples of 30 g of mineral were taken from each tray in order to analyze soluble copper by absorption spectrophotometry. The samples were taken with a spoon, trying to comprise all the strata established through the thickness of the mineral, in order to get a representative sample.

Subsequently, three stages of humidification and dehydration were carried out. The added acid solution in each stage was homogeneously distributed with a sprinkler.

Finally, the mineral was washed keeping it in contact with the solution during six hours to enable eventual indirect increases of copper recovery. Soluble copper was determined in the filtered supernatant and the final extraction was calculated.

Table IV indicates relevant information concerning the operative conditions and required times in each stage, and also the resultant copper extraction from each stage, for the two trays.

Table IV also indicates the volume of the acid solution and the quantity of acid added in each stage per kilogram of mineral. In practice, it was added a proportional volume of acid, corresponding to the real quantity of remanent mineral, taking into account mineral samples previously removed.

It could be seen that operating under the conditions indicated in Table IV it is possible to achieve a copper extraction between 66 and 68% in about three days. It is known that with conventional systems operating at similar scales, the necessary time to reach equivalent extraction yields ranges from seventy to ninety days.

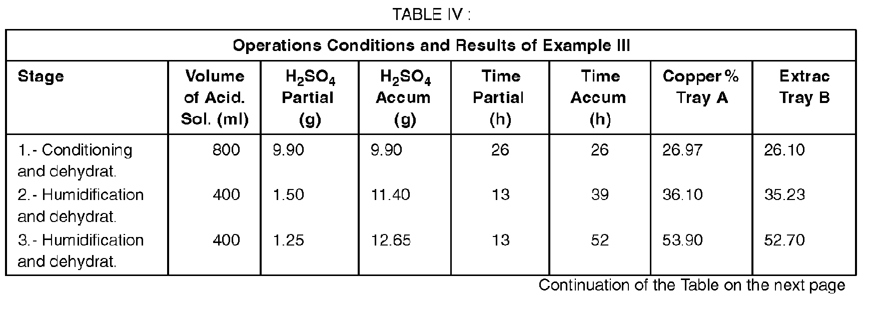

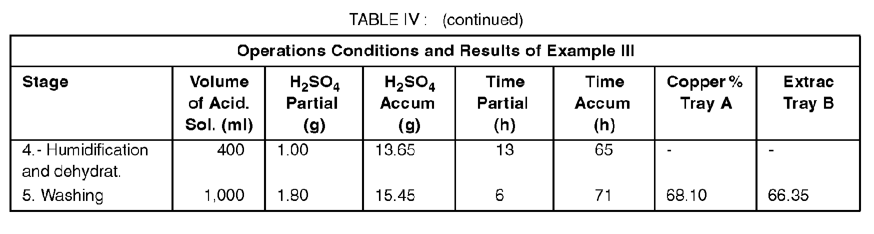

## Claims

1. A bio-metallurgical process in which mineral compounds contained in mineral ores or concentrates and constituting substrates for microorganisms, arc bio-oxidizcd, dissolved and separated, characterized by the following stops:
a) conditioning the mineral ore or the concentrate with the quantity of acid which is determined before-hand as the minimum volume that ensures the total and homogenous acidification of the substrate and having a concentration which ensures neutralization of the mineral ore or the concentrate, prevents compaction and provides an environment for microbial development, or contacting the mineral ore or the concentrate with acid vapours so as to homogeneously acidify the substrate while introducing the minimum possible amount of water required to provide an effective coating of the mineral ore or concentrate into the system;
b) adding a microbial inoculum capable of oxidating the mineral compound of interest, or enriching the mineral ore's own microbial flora, either simultaneously with or independently from step a);
c) enabling the spontaneous or induced loss of the excess of water that may be present in the system by evaporation or by dehydrating with flowing air until the thermodynamically available water is sufficiently low for obtaining the bio-oxidation products in solid state, said bio-oxidation products, in turn, constituting microbial colonies; and
d) separating the bio-oxidation products.

2. A process according to claim 1, characterized in that said quantity of acid is contained in the minimum possible volume of a solution that ensures the total and homogenous acidification of the mineral ore or concentrate

3. A process according to claim 1, characterized in that the conditioning of mineral ore or concentrate includes the addition of compounds in the form of a salt or an aqueous solution comprising at least one element selected from potassium, phosphorous, magnesium, calcium and nitrogen, for enabling the microbial development and bio-oxidation of the substrate, such addition depending on the microbial strain and the composition of the mineral ore or concentrate in question.

4. A process according to Claim 1, characterized in that the volume of the conditioning solution is diminished by the addition of surfactants which also improve microbial development.

5. A process according to Claim 1, characterized in that bio-oxidation activity is enhanced by decreasing the thermodynamically available water by the addition of a dehydrating agent.

6. A process according to Claim 1, characterized in that the microorganisms used therein develop at temperatures ranging from 5 to 100°C.

7. A process according to Claim 1, characterized in that the thermodynamically available water is increased and decreased alternately in order to solubilize at least partially the bioxidized particle thus releasing the microorganims contained therein, whereby said microorganisms, in a humid environment, reach new substrate particles and oxidize these particles after the subsequent decrease of water activity, and repeating these operations as required to obtain the expected yield.

8. A process according to Claim 1, characterized in that the bio-oxidation products are separated by washing or screening.

9. A process according to Claim 8, characterized in that in the bioleaching of metals, the bio-oxidized compounds will be the products of interest and the remaining solids will constitute the residuum, and in purification processes, the bio-oxydized compounds will be the residuum and the remaining solids will constitute the purified product.

## Patentansprüche

1. Biometallurgisches Verfahren, bei dem in Mineralerzen oder -konzentraten enthaltene mineralische Verbindungen, die Substrate für Mikroorganismen darstellen, biooxidiert, gelöst und abgetrennt werden, charakterisiert durch die folgenden Schritte:
a) Konditionieren des Mineralerzes oder des Konzentrats mit der Menge an Säure, die zuvor als das Minimalvolumen bestimmt wird, das die gesamte und homogene Ansäuerung des Substrats sicherstellt mit einer Konzentration, die die Neutralisierung des Mineralerzes oder des Konzentrats sicherstellt, Kompaktieren verhindert und eine Umgebung für mikrobielle Entwicklung schafft,
oder Kontaktieren des Mineralerzes oder des Konzentrats mit Säuredämpfen zur homogenen Ansäuerung des Substrats, wobei die geringstmögliche Menge an Wasser in das System eingeführt wird, die erforderlich ist, um eine wirksame Beschichtung des Mineralerzes oder -konzentrats bereitzustellen;
b) Eintragen eines mikrobiellen Inoculums, das fähig ist zum Oxidieren der interessierenden mineralischen Verbindung, oder zum Anreichern der dem Mineralerz eigenen mikrobiellen Flora, entweder simultan mit oder unabhängig von Schritt a);
c) Ermöglichen des spontanen oder induzierten Verlustes des Überschußwassers, das im System vorliegen kann, durch Verdampfen oder durch Dehydratisieren im Luftstrom, bis das thermodynamisch verfügbare Wasser ausreichend wenig ist, um die Bio-oxidationsprodukte in festem Zustand zu erhalten, wobei die Biooxidationsprodukte ihrerseits mikrobielle Kolonien darstellen; und
d) Abtrennen der Bio-Oxidationsprodukte.

2. Vorfahren nach Anspruch 1, dadurch gekennzeichnet, daß die Menge an Säure im kleinstmöglichen Volumen einer Lösung enthalten ist, die die gesamte und homogene Ansäuerung des Mineralerzes oder -konzentrats sicherstellt.

3. Vorfahren nach Anspruch 1, dadurch gekennzeichnet, daß das Konditionieren des Mineralerzes oder -konzentrats das Eintragen von Verbindungen in Form eines Salzes oder einer wäßrigen Lösung einschließt, umfassend mindestens ein Element, ausgewählt aus Kalium, Phosphor, Magnesium, Calcium und Stickstoff zum Ermöglichen der mikrobiellen Entwicklung und Biooxidation des Substrats, wobei das Eintragen abhängt vom mikrobiellen Stamm und der Zusammensetzung des betreffenden Mineralerzes oder -konzentrats.

4. Verfahren nach Anspruch 1, dadurch gekennzeichnet, daß das Volumen der Konditionierungslösung vermindert ist durch Versetzen mit oberflächenaktiven Stoffen, die auch die mikrobielle Entwicklung verbessern

5. Verfahren nach Anspruch 1, dadurch gekennzeichnet, daß die Biooxidationsaktivität erhöht wird durch Vermindern des thermodynamisch verfügbaren Wassers durch Versetzen mit einem Dehydratisierungsmittel.

6. Verfahren nach Anspruch 1, dadurch gekennzeichnet, daß sich die verwendeten Mikroorganismen bei Temperaturen von 5 bis 100 °C entwickeln.

7. Verfahren nach Anspruch 1, dadurch gekennzeichnet, daß das thermodynamisch verfügbare Wasser abwechselnd vermehrt und vermindert wird, um die biooxidierten Partikel zumindest teilweise löslich zu machen und somit die darin enthaltenen Mikroorganismen freizusetzen, wobei die Mikroorganismen in einer feuchten Umgebung neue Substratteilchen erreichen und diese Teilchen nach der darauffolgenden Verminderung der Wasseraktivität oxidieren, und diese Verfahrensschritte wiederholt werden, wie es erforderlich ist, um die gewünschte Ausbeute zu erhalten

8. Verfahren nach Anspruch 1, dadurch gekennzeichnet, daß die Biooxidationsprodukte durch Waschen oder Sieben/Filtrieren abgetrennt werden.

9. Verfahren nach Anspruch 8, dadurch gekennzeichnet, daß bei Bioleaching von Metallen die biooxidierten Verbindungen die interessierenden Produkte sind und die verbleibenden Feststoffe den Rückstand darstellen, und in Reinigungsverfahren die biooxidierten Verbindungen den Rückstand darstellen und die verbleibenden Feststoffe das gereinigte Produkt darstellen.

## Revendications

1. Un processus bio-métallurgique dans lequel on bio-oxyde, on dissout et on sépare des composés minéraux contenus dans des minerais minéraux ou concentrés et constituant des substrats pour des micro-organismes, caractérisé par les étapes consistant à :
a) conditionner le minerai minéral ou le concentré avec la quantité d'acide qui est déterminée au préalable comme le volume minimal qui assure l'acidification totale et homogène du substrat et qui présente une concentration qui assure la neutralisation du minerai minéral ou du concentré, empêche le tassement et procure un environnement pour le développement microbien, ou mettre en contact le minerai minéral ou le concentré avec des vapeurs acides de manière à acidifier de manière homogène le substrat tout en introduisant la quantité minimale possible d'eau requise pour assurer un revêtement efficace du minerai minéral du concentré vers le système ;
b) ajouter un inoculant microbien capable d'oxyder le composé minéral concerné, ou enrichir la flore microbienne propre du minerai minéral, soit simultanément soit indépendamment de l'étape a) ;
c) permettre la perte spontanée ou induite de l'excès d'eau susceptible d'être présente dans le système par évaporation ou par déshydratation par de l'air en mouvement jusqu'à ce que l'eau thermodynamiquement disponible soit suffisamment basse pour obtenir les produits de bio-oxydation à l'état solide, ces produits de bio-oxydation, quant à eux, constituant des colonies microbiennes ; et
d) séparer les produits de bio-oxydation.

2. Un processus selon la revendication 1, caractérisé en ce que ladite quantité d'acide est contenue dans le volume minimum possible d'une solution qui assure l'acidification totale et homogène du minerai minéral ou du concentré.

3. Un processus selon la revendication 1, caractérisé en ce que le conditionnement du minerai minéral du concentré inclut l'addition de composés sous forme d'un sel ou d'une solution aqueuse comprenant au moins un élément choisi parmi le potassium, le phosphore, le magnésium, le calcium et l'azote, pour permettre le développement microbien et la bio-oxydation du substrat, cette addition dépendant de la souche microbienne et de la composition du minerai minéral ou du concentré concerné.

4. Un processus selon la revendication 1, caractérisé en ce que le volume de la solution de conditionnement est réduit par addition de tensio-actifs qui améliorent également le développement microbien.

5. Un processus selon la revendication 1, caractérisé en ce que l'activité de bio-oxydation est renforcée en réduisant l'eau thermodynamiquement disponible par addition d'un agent déshydratant

6. Un processus selon la revendication 1, caractérisé en ce que les micro-organismes qui y sont utilisés se développent à des températures allant de 5 à 100 °C.

7. Un processus selon la revendication 1, caractérisé en ce que l'eau thermodynamiquement disponible est alternativement augmentée et réduite afin de solubiliser au moins partiellement les particules bio-oxydées en libérant ainsi les micro-organismes qui y sont contenus, de manière que ces micro-organismes, dans un environnement humide, atteignent de nouvelles particules du substrat et oxydent ces particules après la réduction subséquente de l'activité de l'eau, et on répète ces opérations en tant que de besoin pour obtenir le rendement attendu.

8. Un processus selon la revendication 1, caractérisé en ce que les produits de bio-oxydation sont séparés par lavage ou criblage.

9. Un processus selon la revendication 8, caractérisé en ce que, dans la bio·lixiviaton des métaux, les composés bio-oxydés seront les produits concernés et les solides restants constitueront le résidu, et dans les processus de purification, les composés bio-oxydés seront le résidu et les solides restants constitueront le produit purifié.
